(19)

Europäisches
Patentamt
European
Patent Office
Office européen
des brevets

(11) **EP 4 414 389 A1**

(12) **EUROPEAN PATENT APPLICATION**
published in accordance with Art. 153(4) EPC

(43) Date of publication:
**14.08.2024 Bulletin 2024/33**

(21) Application number: **22878718.0**

(22) Date of filing: **24.08.2022**

(51) International Patent Classification (IPC):
**C08B 11/12** *(2006.01)* **C08B 15/10** *(2006.01)*
**C08J 3/28** *(2006.01)* **C08J 3/24** *(2006.01)*
**C08L 1/28** *(2006.01)* **C08G 69/10** *(2006.01)*
**C08L 77/04** *(2006.01)*

(52) Cooperative Patent Classification (CPC):
**C08B 11/12; C08B 15/04; C08B 15/10;
C08G 69/10; C08J 3/24; C08J 3/28; C08L 1/28;
C08L 77/04**

(86) International application number:
**PCT/KR2022/012619**

(87) International publication number:
**WO 2023/058897 (13.04.2023 Gazette 2023/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.10.2021  KR 20210131911
05.10.2021  KR 20210131912
05.10.2021  KR 20210131913
08.03.2022  KR 20220029366
08.03.2022  KR 20220097897**

(71) Applicant: **Inertia Inc.
Daejeon 34051 (KR)**

(72) Inventors:
• **KIM, Hyo Yi**
  **Daejeon 34051 (KR)**
• **PARK, Ji Hye**
  **Daejeon 34051 (KR)**
• **KO, Eun Bie**
  **Daejeon 34051 (KR)**
• **LEE, Seung Min**
  **Daejeon 34051 (KR)**

(74) Representative: **Roos, Peter**
  **c/o Roospatent**
  **Noldinstrasse 7**
  **81545 München (DE)**

(54) **ECO-FRIENDLY SUPER ABSORBENT CARBOXYMETHYL CELLULOSE POLYMER AND PREPARATION METHOD THEREFOR**

(57)    The present invention relates to an eco-friendly super-absorbent carboxymethyl cellulose polymer and a preparation method therefor. The eco-friendly super-absorbent carboxymethyl cellulose polymer of the present invention is prepared by crosslinking carboxymethyl cellulose using an electron beam and thus has excellent biodegradability. The polymer is free of toxic materials or microplastics and exhibits high absorbency and thus is suitable for use in sanitary products such as sanitary pads and diapers.

Fig. 1

| | |
|---|---|
| CMC and water mixing step | S10 |
| Electron beam irradiating step | S20 |
| Drying step | S30 |
| Pulverizing step | S40 |

**EP 4 414 389 A1**

**Description**

BACKGROUND

**1. Field of the Invention**

The present invention relates to an eco-friendly super absorbent carboxymethyl cellulose polymer and a preparation method therefor, and specifically, to an eco-friendly super absorbent carboxymethyl cellulose resin, which is prepared by crosslinking carboxymethyl cellulose using an electron beam and thus has excellent biodegradability, and is free of toxic materials or microplastics and exhibits high absorbency, and thus is suitable for use in sanitary products such as sanitary pads and diapers, and a preparation method therefor.

**2. Discussion of Related Art**

A super absorbent polymer (SAP), which is currently used in the field of sanitary products such as sanitary pads or diapers, is a synthetic polymer material that has the function capable of absorbing moisture tens of times its own weight. Since such super absorbent polymers started to be put into practical use in sanitary products such as female sanitary pads and pediatric diapers, now they have been widely used for water retaining soil products for gardening, water stop materials for the civil engineering and construction, sheets for raising seedling, fresh-keeping agents for food distribution fields, and materials for poultice and the like.

However, commercially available SAPs are prepared from petroleum-based resins such as acrylic and ethylene resins, but such petroleum-based resins are toxic, and thus may cause irritation or disease to a user due to the characteristics of sanitary products that come into direct contact with the skin on sensitive areas, and are not biodegradable, causing environmental problems.

Although there have been attempts to prepare sanitary products using eco-friendly materials such as natural super absorbents in order to solve these problems, the natural super absorbents have problems in that it is difficult to apply them to sanitary products or process efficiency significantly deteriorates because they have lower absorption rates than SAPs made of conventional petroleum-based resins or cannot maintain appropriate strength, and have a problem in that they are not sufficiently biodegradable particularly even though they are eco-friendly materials.

SUMMARY OF THE INVENTION

Accordingly, a problem to be solved by the present invention is to provide an eco-friendly super absorbent carboxymethyl cellulose polymer with significantly excellent biodegradability by crosslinking carboxymethyl cellulose using an electron beam, and a preparation method therefor.

Further, another problem to be solved by the present invention is to provide an eco-friendly carboxymethyl cellulose polymer which is suitable for use in sanitary products such as sanitary pads and diapers by replacing existing SAPs, and a preparation method therefor because the eco-friendly super absorbent carboxymethyl cellulose polymer is free of toxic materials or microplastics, has high absorbency, and also has excellent process efficiency.

The problems of the present invention are not limited to the aforementioned technical problems, and other technical problems, which have not been mentioned, may be clearly understood by a person with ordinary skill in the art from the following description.

To solve the above problems, a method for preparing an eco-friendly super absorbent carboxymethyl cellulose (CMC) polymer according to an embodiment of the present invention includes:

(a) mixing 5 to 25 wt% of a CMC with a viscosity of 1,000 cps or more and 75 to 95 wt% of water (S10);
(b) irradiating the mixture with an electron beam (S20);
(c) drying the irradiated mixture (S30); and
(d) pulverizing the dried mixture (S40).

In another embodiment, the method for preparing an eco-friendly super absorbent CMC polymer may further include:

(e) subjecting the mixture pulverized through step (d) above to secondary drying (S32); and
(f) subjecting the secondary dried mixture to secondary pulverization (S42).

Specifically, when the moisture of the mixture is dried by 40 to 60 wt% in step (c) (primary drying), the mixture is pulverized through step (d) (primary pulverization) and completely dried through step (e), and then may be subjected to secondary pulverization through step (f).

The drying temperature in step (e) may be the same as or different from that in step (c), and may be independently selected.

The CMC may have a viscosity of 1,000 or more, preferably 1,500 or more, 2,000 or more, 2,500 or more, or 3,000 or more. The CMC may have a viscosity of more preferably 1,000 to 10,000 cps, 1,500 to 10,000 cps, 2,000 to 10,000 cps, 2,500 to 10,000 cps, 3,000 to 10,000 cps, 1,000 to 9,000 cps, 1,000 to 8,000 cps, 1,000 to 7,000 cps, 1,000 to 6,000 cps, 1,000 to 5,000 cps, 1,000 to 4,000 cps, 1,000 to 3,000 cps, 1,500 to 9,000 cps, 1,500 to 8,000 cps, 1,500 to 7,000 cps, 1,500 to 6,000 cps, 1,500 to 5,000 cps, 1,500 to 4,000 cps, 1,500 to 3,000 cps, 2,000 to 9,000 cps, 2,000 to 8,000 cps, 2,000 to 7,000 cps, 2,000 to 6,000 cps, 2,000 to 5,000 cps, 2,000 to 4,000 cps, 2,000 to 3,000 cps, 3,000 to 9,000 cps, 3,000 to 8,000 cps, 3,000 to 7,000 cps, 3,000 to 6,000 cps, 3,000 to 5,000 cps, 3,000 to 4,000 cps, 4,000 to 10,000 cps, 5,000 to 9,000 cps, 6,000 to 8,000 cps, 1,500 to 3,500 cps, 2,000 to 3,500 cps, 2,500 to 4,000 cps, 2,500 to 3,500 cps or 2,500 to 3,000 cps.

When the viscosity of the CMC is less than the above range, the degree of dissociation is greater than the degree of crosslinking during crosslinking by irradiation using an electron beam, so that it may be difficult to prepare a mixture in the form of a gel. When the viscosity exceeds the above range, the economic feasibility may deteriorate, and the strength of the gel becomes excessively high, so that it may be difficult to pulverize the mixture.

The irradiation dose of the electron beam in step (b) above may be 10 to 40 kGy, 15 to 40 kGy, 20 to 40 kGy, 10 to 35 kGy, 10 to 30 kGy, 15 to 30 kGy, 20 to 30 kGy, 10 to 25 kGy, 15 to 25 kGy, or 20 to 25 kGy. The irradiation dose of the electron beam may be determined by the dose per hour and the irradiation time, and in this case, the irradiation time may be 0.1 seconds to 1 minute, but is not limited thereto.

When a mixture of CMC and water is irradiated with an electron beam, a crosslinking reaction of CMC occurs, so that the mixture is gelled, but when the irradiation dose is too high, gelation and curing may become excessive, whereas when the irradiation dose is too low, proper gelation and curing are not induced. In addition, the irradiation dose of the electron beam also affects the biodegradability and pulverization yield of the CMC polymer, but the higher the degree of crosslinking, the lower the biodegradability and pulverization yield tend to be.

Therefore, when the irradiation dose of the electron beam is within the above range, it is possible to obtain the best biodegradability and effective gel strength and pulverization yield, which may be calculated using the parameters as described below.

The drying temperature in step (c) may be 10 to 70°C, preferably 10 to 60°C, more preferably 20 to 50°C, and even more preferably 20 to 40°C. Alternatively, step (c) above may be a freeze-drying step.

When the temperature at which the crosslinked CMC mixture is dried is low, it is advantageous for biodegradability and pulverization yield, but the drying time is elongated, so that the process efficiency is reduced, whereas the higher the temperature is, the shorter the drying time is, so that the process efficiency is increased, but biodegradability and pulverization yield may be reduced.

Therefore, when the drying temperature is within the above range, it is possible to obtain the best biodegradability and effective time taken for drying and pulverization yield, which may be calculated using the parameters as described below.

The CMC polymer pulverized after being dried may be screened through a sieve and the like to obtain a desired particle size. The average particle size of the screened CMC polymer may be 100 $\mu$m to 1 mm, but is not limited thereto. When the average particle size of the screened CMC polymer is out of the above range, the basic functionality or usability of products to which the CMC polymer is applied may deteriorate.

The prepared CMC polymer may have gel strength, biodegradability and pulverization yield with a dose dependent utility value ($F_1$) of 2 or more, preferably 2.1 or more, more preferably 2.2 or more, and even more preferably 2.3 or more calculated by the following Equation 1-1.

Alternatively, the irradiation dose of the electron beam may be a dose, such that the prepared CMC polymer has a utility value of 2 or more, preferably 2.1 or more, more preferably 2.2 or more, and even more preferably 2.3 or more. The irradiation dose of the electron beam, which satisfies the above range, may be 10 to 40 kGy, preferably 10 to 35 kGy, and more preferably 10 to 30 kGy.

$$[\text{Equation 1-1}]$$

$$F_1 = X_1 + Y + Z$$

In Equation 1-1, for each of $X_1$, Y and Z,

i) $X_1 = 0$ (when the gel strength is 0 kPa or more and less than 20 kPa)

$$X_1 = 0.2 + (x/40)^{\wedge}2$$

(X is the gel strength; when the gel strength is 20 kPa or more and less than 40 kPa) or
$X_1$ = 1.2 (when the gel strength is 40 kPa or more)
ii) $Y = y^{\wedge}2$ (y is the biodegradability)
iii) $Z = z$ (z is the pulverization yield).

The gel strength may be a value obtained by swelling a gel, which is a crosslinked CMC mixture, in distilled water to saturation for 1 hour, and then measuring the strength of the swollen gel using a universal physical property measuring instrument.

The biodegradability may be calculated as shown in the following Equation 2-1.

Biodegradability = 1 - (the dry mass (g) of an absorbent filtered by a sieve with a size of 350 $\mu$m 10 days after 1 g of the absorbent with a particle size of 500 $\mu$m or more is swollen in water and buried in soil filtered by a sive with a size of 350 $\mu$m or less)     [Equation 2-1]

The pulverization yield may be calculated as shown in the following Equation 2-2.

Pulverization yield = {(input mass - amount of fine powder (=<350 $\mu$m) based on pulverization of a roll mill once and a pin mill eqipped with a sieve with a size of 850 $\mu$m once) + loss due to carbonization} / input mass     [Equation 2-3]

The above gel strength, biodegradability, and pulverization yield may be values obtained under conditions where the drying temperature is fixed at 40°C, but are not limited thereto.

On the other hand, in a non-limiting embodiment, the gel strength may be 20 to 80 kPa, preferably 25 to 75 kPa, more preferably 25 to 55 kPa. The biodegradability may be 0.5 to 0.9, preferably 0.6 to 0.9, more preferably 0.6 to 0.85. The pulverization yield may be 0.4 to 0.98, preferably 0.5 to 0.98, more preferably 0.7 to 0.97.

When the irradiation dose is 18 to 40 kGy, the absorption rate of the CMC polymer may have the best value at 5,000% or more, preferably 5,100% or more, more preferably 5,200% or more for 1 wt% of brine. Therefore, the irradiation dose that satisfies the above dose dependent utility value to the maximum and simultaneously has the best absorption rate may be 18 to 40 kGy, preferably 18 to 35 kGy, more preferably 18 to 30 kGy.

The prepared CMC polymer may have time taken for drying, biodegradability and pulverization yield with a temperature dependent utility value ($F_2$) of 2.2 or more, preferably 2.4 or more, more preferably 2.45 or more, and even more preferably 2.5 or more calculated by the following Equation 1-2.

Alternatively, the drying temperature may be a temperature, such that the prepared CMC polymer has a utility value of 2.2 or more, preferably 2.4 or more, more preferably 2.45 or more, and even more preferably 2.5 or more. The drying temperature satisfying this requirement may be 10 to 70°C, preferably 10 to 60°C, more preferably 20 to 50°C, and even more preferably 20 to 40°C.

[Equation 1-2]

$$F_2 = X_2 + Y + Z$$

In Equation 1-2, for each of $X_2$, Y and Z,

i) $X_2$ = 1.2 (when the time taken for drying is 0 hour or more and less than 24 hours)
$X_2$ = 1 (when the time taken for drying is 24 hours or more and less than 48 hours)
$X_2$ = 0.9 (when the time taken for drying is 48 hours or more and less than 72 hours)
$X_2$ = 0.7 (when the time taken for drying is 72 hours or more and less than 96 hours) or
$X_2$ = 0.3 (when the time taken for drying is 96 hours or more)
ii) $Y = y^{\wedge}2$ (y is the biodegradability)
iii) $Z = z$ (z is the pulverization yield).

The time taken for drying may be the time it takes for the moisture content of a dry material to reach less than 10% based on a 400 x 800 mm tray of 5kg of an original material.

The biodegradability and pulverization yield may be calculated as shown in Equations 2-1 and 2-2 above.

The above time taken for drying, biodegradability, and pulverization yield may be values obtained under conditions where the irradiation dose of the electron beam is fixed at 20 kGy, but are not limited thereto.

On the other hand, in a non-limiting embodiment, the time taken for drying may be 30 to 85 hours, preferably 35 to 85 hours, more preferably 40 to 75 hours. The biodegradability may be 0.6 to 0.9, preferably 0.7 to 0.9, more preferably 0.7 to 0.85. The pulverization yield may be 0.8 to 0.99, preferably 0.85 to 0.99, more preferably 0.9 to 0.98.

The method for preparing the CMC polymer of the present invention and the prepared CMC polymer may not use or contain additives other than water and CMC. For example, by not containing an additive such as starch, citric acid, a carboxymethylation agent, and a catalyst, the CMC polymer may have absorption rate, gel strength, and the like suitable for use in sanitary products, and the like while maximizing eco-friendliness, and the absorption rate, gel strength, and the like may be implemented using the above-described utility value, and the like.

The prepared CMC polymer may satisfy the physical properties of one or more, preferably two or more, and more preferably three of the following (1) to (3).

(1) Absorption rate (%): 2,000 or more, preferably 2,500 or more, more preferably 3,000 or more, more preferably 3,500 or more, more preferably 4,000 or more, more preferably 4,500 or more, and even more preferably 5,000 or more

(2) Loss on drying (%): 10% or less, preferably 8% or less, more preferably 5% or less, and even more preferably 4% or less

(3) Residue on ignition (%): 14 to 28, preferably 18 to 27, more preferably 20 to 26, and even more preferably 22 to 26

Alternatively, the prepared CMC polymer may satisfy one or more detection amount characteristics of the following (1) and (2).

(1) one or more of cadmium (Cd), mercury (Hg), lead (Pd), hexane, chloroform, benzene, trichloroethylene, toluene, tetrachloroethylene, ethylbenzene, xylene and styrene: Not detected

(2) Microplastics (45 $\mu$m or more): Not detected

Alternatively, the prepared CMC polymer may satisfy two or more, preferably four or more, more preferably six or more, and even more preferably ten or more test criteria of the following (1) to (14).

(1) Confirmation test (cellulose gum) 1: becomes a viscous liquid

(2) Confirmation test (cellulose gum) 2: a white precipitate is produced

(3) Sodium salt (confirmed): appears yellow in color

(4) Sodium salt (confirmed) 2: a white crystalline precipitate is produced

(5) Confirmation test (cellulose gum) 3: a fine white precipitate is produced

(6) Confirmation test (cellulose gum) 4: the liquid appears reddish-purple in color

(7) Purity test (dissolved state): an average value obtained by repeating the test three times is greater than an average value obtained by performing the same operation using the following comparison solution.

(8) Purity test (alkali): the liquid does not appear light red in color

(9) Purity test (chloride): the turbidity exhibited by the test solution is not more pronounced than the turbidity exhibited by the comparison solution.

(10) Purity test (chloride): the turbidity exhibited by the test solution is not more pronounced than the turbidity exhibited by the comparison solution

(11) Purity test (silicate) (%): 1.5 or less, preferably 1 or less, more preferably 0.6 or less

(12) Purity test (heavy metal): the color exhibited by the test solution is not darker than the color exhibited by the comparison solution and/or 20 ppm or less

(13) Purity test (arsenic): the test solution is not darker than the standard color and/or less than 2 ppm

(14) Purity test (starch): the liquid does not appear blue in color

Specific details of other embodiments are included in the detailed description.

BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

FIG. 1 is a flowchart showing a method for preparing an eco-friendly super absorbent carboxymethyl cellulose (CMC) polymer according to an example of the present invention;

FIG. 2 is a flowchart showing a method for preparing a CMC polymer according to another example of the present invention;

FIG. 3 is a graph showing the gel strength and the like of a CMC polymer prepared according to an experimental example of the present invention;

FIG. 4 is a graph showing the utility value of a CMC polymer prepared according to an experimental example of the present invention;

FIG. 5 is a graph showing the time taken for drying and the like of a CMC polymer prepared according to an experimental example of the present invention;

FIG. 6 is a graph showing the utility value of a CMC polymer prepared according to an experimental example of the present invention;

FIGS. 7 to 9 are scanning electron microscope (SEM) images of CMC polymers dried at a low temperature, 40°C, and a high temperature, respectively, according to an experimental example of the present invention; and

FIG. 10 is a graph showing the absorption rate of a CMC polymer prepared according to an experimental example of the present invention.

DETAILED DESCRIPTION OF EXEMPLARY EMBODIMENTS

The benefits and features of the present invention, and the methods of achieving the benefits and features will become apparent with reference to embodiments to be described below in detail. However, the present invention is not limited to the embodiments to be disclosed below and may be implemented in various other forms, and the embodiments are only provided for rendering the disclosure of the present invention complete and for fully representing the scope of the invention to a person with ordinary skill in the technical field to which the present invention pertains, and the present invention will be defined only by the scope of the claims.

The terms used in the present specification are used merely to describe embodiments, and are not intended to limit the present invention. In the present specification, the 'and/or' includes each and all combinations of one or more of the items mentioned. Further, the singular form also includes the plural forms unless specifically stated in a phrase. The terms 'comprises' and/or 'comprising' used in the specification do not exclude the presence or addition of one or more other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

Further, in describing the constituent elements of the examples of the present invention, terms such as first, second, A, B, (a), and (b) may be used. These terms are merely for distinguishing one constituent element from another, and the nature, turn, or order of the corresponding constituent element is not limited by the term.

Unless otherwise defined, all the terms (including technical and scientific terms) used in the present specification will be able to be used as a meaning which may be commonly understood to a person with ordinary skill in the art to which the present invention pertains. In addition, the terms defined in generally used dictionaries are not to be interpreted ideally or excessively unless clearly and specifically defined.

Moreover, in describing the examples of the present invention, when it is determined that the specific description of relevant known configurations or functions obstructs the understanding for the examples of the present invention, the detailed description thereof will be omitted.

Hereinafter, the present invention will be described through Preparation Examples and Experimental Examples, but it is obvious that the effects of the present invention are not limited by the following Experimental Examples.

**Preparation Example 1**

After 10 wt% of a CMC with a viscosity of 1500 cps was mixed with 90 wt% of water, a plurality of samples were irradiated with different doses of electron beams as shown in the following Table 1 to induce a crosslinking reaction. The crosslinked mixture was dried at 40°C until being completely dried, and then pulverized to prepare particles with an appropriate particle size. Alternatively, the crosslinked mixture was half dried at 40°C, pulverized into large particles by performing primary pulverization, completely dried by performing secondary drying, and then subjected to secondary pulverization.

[Table 1]

| Example | Irradiation dose (kGy) |
|---------|------------------------|
| Example 1-1 | 0 |

(continued)

| Example | Irradiation dose (kGy) |
|---|---|
| Example 1-2 | 5 |
| Example 1-3 | 10 |
| Example 1-4 | 15 |
| Example 1-5 | 20 |
| Example 1-6 | 25 |
| Example 1-7 | 30 |
| Example 1-8 | 35 |
| Example 1-9 | 40 |
| Example 1-10 | 45 |
| Example 1-11 | 50 |

**Experimental Example 1**

The following items were evaluated for the prepared CMC polymers of Examples 1-1 to 1-11.

- Gel strength: obtained by swelling a gel, which is a crosslinked CMC mixture, in distilled water to saturation for 1 hour, and then measuring the strength of the swollen gel using a universal physical property measuring instrument
- Biodegradability: 1 - (the dry mass (g) of an absorbent filtered by a sieve with a size of 350 $\mu$m 10 days after 1 g of the absorbent with a particle size of 500 $\mu$m or more is swollen in water and buried in soil filtered by a sieve with a size of 350 $\mu$m or less)
- Pulverization yield: {(input mass - amount of fine powder (=< 350 $\mu$m) based on pulverization of a roll mill once and a pin mill equipped with a sieve with a size of 850 $\mu$m once) + loss due to carbonization} / input mass

A dose dependent utility value ($F_1$) was obtained using a value obtained by the method as described above in the following Equation 1-1.

$$[\text{Equation 1-1}]$$

$$F_1 = X_1 + Y + Z$$

In Equation 1-1, for each of $X_1$, Y and Z,

i) $X_1 = 0$ (when the gel strength is 0 kPa or more and less than 20 kPa)
$X_1 = 0.2 + (x/40)^2$ (x is the gel strength; when the gel strength is 20 kPa or more and less than 40 kPa) or
$X_1 = 1.2$ (when the gel strength is 40 kPa or more)
ii) $Y = y^2$ (y is the biodegradability)
iii) $Z = z$ (z is the pulverization yield).

When sanitary products such as sanitary pads are prepared using a CMC polymer with a gel strength of 0 to 20 kPa, the sanitary products are not economically useful because there is a big risk in that when pressure is applied while a user is in a posture such as sitting on a chair, the gel in the product loses its shape and comes into contact with the skin. Therefore, $X_1$, which is a gel strength function, may be defined as 0 when the gel strength ($x_1$) is 0 kPa or more and less than 20 kPa.

When the gel strength is 20 kPa, the CMC polymer may have utility in which the minimum utility, $X_1$ ($x_1$), may be defined as 0.2, and as the gel strength increases (up to 40 kPa), the usable range (sales range) increases in a squared manner, and in this case, the gel strength may be divided by 40, which is the maximum value of the range, for normalization.

When the gel is cured as the gel strength is 40 kPa or more, the range in which the gel is used does not vary even when the gel strength increases, and $X_1$ ($x_1$) may be defined as having a fixed value of 1.2, which is 6 times more useful than the minimum utility gel strength of 20 kPa.

As the biodegradability of the CMC polymer increases, the economic feasibility obtained from reduced costs may be defined as increasing in a squared manner. Therefore, Y, which is a biodegradability function, may be expressed as the square of the biodegradability (y).

The pulverization yield of the CMC polymer is the proportion of an effective material obtained after pulverizing the CMC polymer, and thus is directly proportional to economic feasibility. Therefore, Z, which is a pulverization yield function, is the same as the pulverization yield (z).

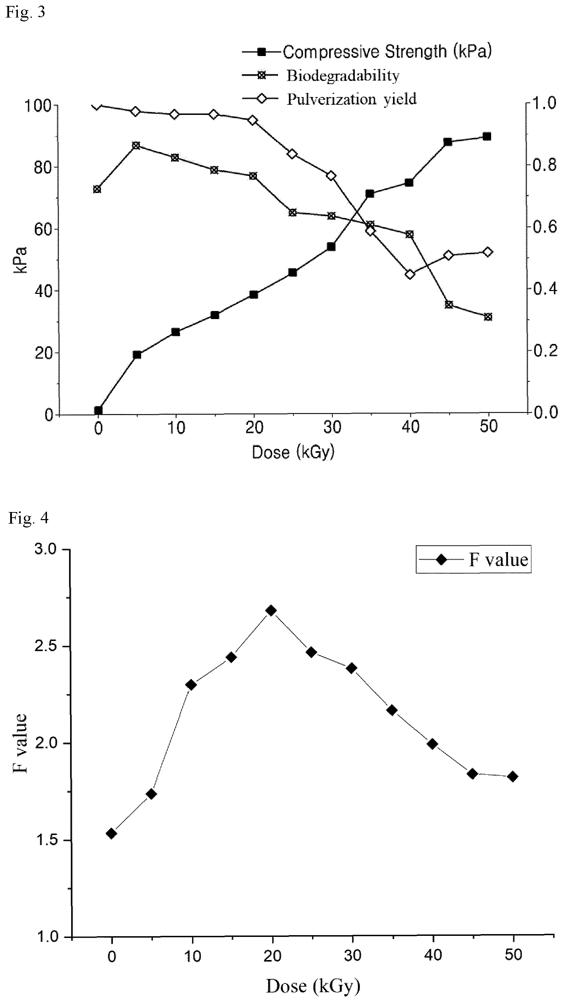

FIG. 3 shows the gel strength, biodegradability, and pulverization yield according to the irradiation dose, and FIG. 4 shows the dose dependent utility value according to the irradiation dose using functions for gel strength, biodegradability, and pulverization yield.

As shown in FIG. 4, when the irradiation dose was out of the suitable range, the dose dependent utility value ($F_1$) decreased rapidly. In other words, it can be seen that the dose dependent utility value ($F_1$) is a novel parameter capable of searching for the most efficient value which means the best result by quantifying the effects of gel strength, biodegradability, and pulverization yield on the actual process and product characteristics as appropriate parameters.

**Preparation Example 2**

After 10 wt% of a CMC with a viscosity of 1,500 cps was mixed with 90 wt% of water, a crosslinking reaction was induced by irradiating the mixture with a 20 kGy electron beam. A plurality of crosslinked samples were dried at different temperatures until being completely dried as shown in the following Table 2, and then pulverized to prepare particles with an appropriate particle size.

[Table 2]

| Example | Drying temperature (°C) |
|---|---|
| Example 2-1 | 5 |
| Example 2-2 | 10 |
| Example 2-3 | 20 |
| Example 2-4 | 30 |
| Example 2-5 | 40 |
| Example 2-6 | 50 |
| Example 2-7 | 60 |
| Example 2-8 | 70 |
| Example 2-9 | 80 |

**Experimental Example 2**

The following items were evaluated for the prepared CMC polymers of Examples 2-1 to 2-9.

- Time taken for drying: the time it takes for the moisture content of a dry material to reach less than 10% based on a 400 x 800 mm tray of 5kg of an original material
- Biodegradability: the same as in Experimental Example 1
- Pulverization yield: the same as in Experimental Example 1

A temperature dependent utility value ($F_2$) was obtained using a value obtained by the method as described above in the following Equation 1-2.

$$[\text{Equation 1-2}]$$

$$F_2 = X_2 + Y + Z$$

In Equation 1-2, for each of $X_2$, Y and Z,

i) $X_2$ = 1.2 (when the time taken for drying is 0 hour or more and less than 24 hours)
$X_2$ = 1 (when the time taken for drying is 24 hours or more and less than 48 hours)
$X_2$ = 0.9 (when the time taken for drying is 48 hours or more and less than 72 hours)

$X_2 = 0.7$ (when the time taken for drying is 72 hours or more and less than 96 hours) or
$X_2 = 0.3$ (when the time taken for drying is 96 hours or more)
ii) $Y = y^2$ (y is the biodegradability)
iii) $Z = z$ (z is the pulverization yield).

Since the cost structure according to the drying time is the same for one day (24 hours), X2, which is a drying time function, has a box function form with an interval length of 24. Assuming that the maximum utility per day is 1.2, the cost increases by about 16% on day 2, but the utility value that reflects the same is 1. When one more day is increased, the cost increases further by about 10% of the corresponding day, and from day 4 onwards, the cost increases rapidly, and thus becomes four times the cost on day 1.

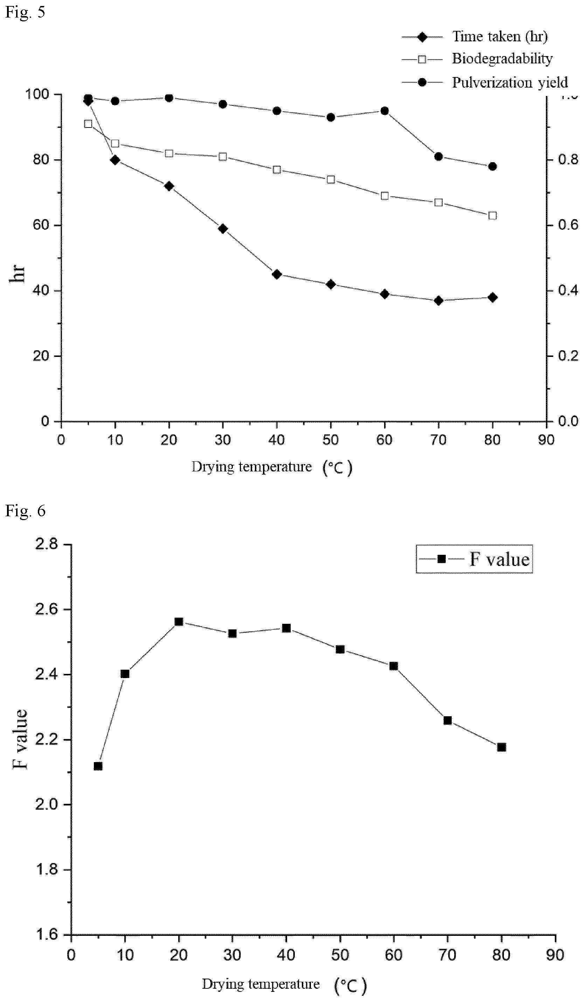

FIG. 5 shows the time taken for drying, biodegradability, and pulverization yield according to the irradiation dose, and FIG. 6 shows the temperature dependent utility value according to the drying temperature using functions for time taken for drying, biodegradability, and pulverization yield.

As shown in FIG. 6, when the drying temperature was out of the suitable range, the temperature dependent utility value ($F_2$) decreased rapidly. In other words, it can be seen that the temperature dependent utility value ($F_2$) is a novel parameter capable of searching for the most efficient value which means the best result by quantifying the effects of time taken for drying, biodegradability, and pulverization yield on the actual process and product characteristics as appropriate parameters.

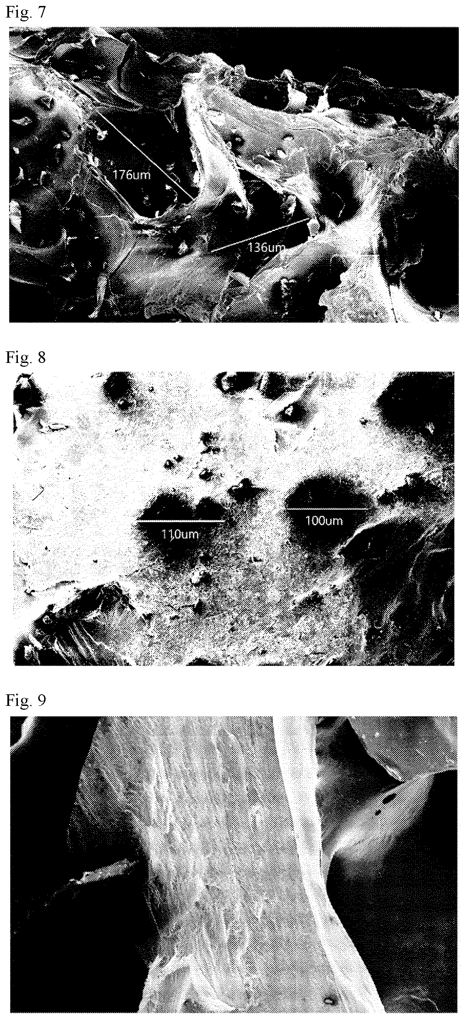

On the other hand, FIGS. 7 to 9 show scanning electron microscope (SEM) images of CMC polymers dried at a low temperature, 40°C, and a high temperature, respectively, and it can be seen that when the drying temperature is not appropriate, the number of pores decreases, and thus, absorbency or biodegradability may deteriorate.

## Preparation Example 3

After 10 wt% of a CMC with a viscosity of 1,500 cps was mixed with 90 wt% of water, a crosslinking reaction was induced by irradiating the mixture with a 20 kGy electron beam. The crosslinked mixture was dried at 40°C until being completely dried, and then pulverized to prepare particles with an appropriate particle size. Alternatively, the crosslinked mixture was half dried at 40°C, pulverized into large particles by performing primary pulverization, completely dried by performing secondary drying, and then subjected to secondary pulverization.

## Experimental Example 3

The items shown in the following Tables 3 and 4 were evaluated for the prepared CMC polymer.

[Table 3]

| Test Item | Test Method |
|---|---|
| Cd | EPA3051A, 6010D (as applicable) |
| Hg | EPA 3051A, Mercury analyzer (as applicable) |
| Pd | EPA 3051A, 6010D (as applicable) |
| Dichloromethane | Gas chromatograph-mass spectrometer (GC/MS) column: 6% cyanopropylphenyl, 94% dimethylpolysiloxane |
| Hexane | |
| Chloroform | |
| Benzene | |
| Trichloroethylene | |
| Toluene | |
| Tetrachloroethylene | |
| Ethylbenzene | |
| Xylene | |
| Styrene | |
| Fluorescent whitening agent | Visual determination method determined under U.V light source (300 nm to 400 nm) |
| Residue on ignition | In a general test method of the Ministry of Food and Drug Safety, a crucible made of platinum, quartz, or porcelain to hold a specimen for a residue on ignition test method is ignited at $600 \pm 50°C$ for 30 minutes in advance, and then left to cool in a desiccator (silica gel or other suitable desiccant), and then the mass thereof is precisely weighed. The amount of a specimen prescribed in each article of pharmaceuticals is measured, the specimen is put into the crucible, and the mass thereof is precisely weighed. A small amount (usually 1 mL) of sulfuric acid is added to the next specimen to soak the specimen, and the specimen is completely carbonized by slowly heating the specimen at a temperature as low as possible. After the specimen is left to cool, the specimen is again soaked with a small amount (usually 1 mL) of sulfuric acid, slowly heated until no white smoke comes out, and again ignited at $600 \pm 50°C$ to completely reduce the residue to ashes. After the crucible is left to cool in a desiccator (silica gel or other suitable desiccant), the mass thereof is precisely weighed to calculate the percentage of the residue. |
| Loss on drying | The Korean Pharmacopoeia: Standards and Test Methods for Quasi-Drugs (KQC) |

(continued)

| Test Item | Test Method |
|---|---|
| Microplastics | ① 100 ml of purified water and 100 ml of ethanol are added to about 3.0 g of a specimen, and the specimen is homogenized by stirring the resulting mixture for at least 10 minutes. ② The homogenized specimen is filtered using a filter for filtration, which is made of a 325 mesh (filter particle diameter: 45 $\mu$m) metal material, and a vacuum filtration device. ③ The filter used for filtration is washed several times with purified water. ④ The filter used for filtration is pinched with metal tweezers, placed on an aluminum plate, covered with aluminum foil, and then dried at 50 to 60°C for 30 minutes or more to completely remove moisture. ⑤ The dried filter for filtration is observed using an infrared spectrometer-microscope (8x magnification) to confirm the presence or absence of foreign material. ⑥ An infrared spectrum is obtained by irradiating observed foreign materials with a size of 5 mm or less with infrared rays using a microscope. ⑦ The spectra are compared with the infrared spectra of plastics to determine whether the plastics are microplastics. |

[Table 4]

| Test Item | Test Method |
|---|---|
| Confirmation test (cellulose gum) 1 | The Korean Pharmacopoeia: Standards and Test Methods for Quasi-Drugs (KQC) |
| Confirmation test (cellulose gum) 2 | |
| Sodium salt (confirmed) | |
| Sodium salt (confirmed) 2 | |
| Confirmation test (cellulose gum) 3 | |
| Confirmation test (cellulose gum) 4 | |
| Purity test (dissolved state) | |
| Purity test (alkali) | |
| Purity test (chloride) | |
| Purity test (sulfate) | |
| Purity test (silicate) (%) | |
| Purity test (heavy metal) | |
| Purity test (arsenic) | |
| Purity test (starch) | |

The evaluation results are shown in the following Tables 5 and 6.

[Table 5]

| Test Item | Test Criteria | Test Result |
|---|---|---|
| Cd (mg/kg) | - | Not detected |
| Hg (mg/kg) | - | Not detected |
| Pd (mg/kg) | - | Not detected |
| Hexane ($\mu$g/g) | - | Not detected |
| Chloroform ($\mu$g/g) | - | Not detected |
| Benzene ($\mu$g/g) | - | Not detected |
| Trichloroethylene ($\mu$g/g) | - | Not detected |
| Toluene ($\mu$g/g) | - | Not detected |
| Tetrachloroethylene ($\mu$g/g) | - | Not detected |
| Ethylbenzene ($\mu$g/g) | - | Not detected |
| Xylene ($\mu$g/g) | - | Not detected |
| Styrene ($\mu$g/g) | - | Not detected |
| Fluorescent whitening agent | - | Not showing fluorescence |
| Residue on ignition (%) | 14.0 to 28.0 | 25.1 |
| Loss on drying (%) | 10 or less | 3.9 |
| Microplastics | 45 $\mu$m or more | Not detected |

[Table 6]

| Test Item | Test Criteria | Test Result |
|---|---|---|
| Confirmation test (cellulose gum) 1 | becomes a viscous liquid. | becomes a viscous liquid |
| Confirmation test (cellulose gum) 2 | A white precipitate is produced. | A white precipitate is produced |
| Sodium salt (confirmed) | appears yellow in color. | appears yellow in color |
| Sodium salt (confirmed) 2 | A white crystalline precipitate is produced. | A white crystalline precipitate is produced |
| Confirmation test (cellulose gum) 3 | A fine white precipitate is produced. | A fine white precipitate is produced |
| Confirmation test (cellulose gum) 4 | The liquid appears reddish-purple in color. | The liquid appears reddish-purple in color |
| Purity test (dissolved state) | an average value obtained by repeating the test three times is greater than an average value obtained by performing the same operation using the following comparison solution. | an average value obtained by repeating the test three times is greater than an average value obtained by performing the same operation using the following comparison solution |
| Purity test (alkali) | The liquid does not appear light red in color. | The liquid does not appear light red in color |
| Purity test (chloride) | The turbidity exhibited by the test solution is not more pronounced than the turbidity exhibited by the comparison solution. | The turbidity exhibited by the test solution is not more pronounced than the turbidity exhibited by the comparison solution |
| Purity test (sulfate) | The turbidity exhibited by the test solution is not more pronounced than the turbidity exhibited by the comparison solution. | The turbidity exhibited by the test solution is not more pronounced than the turbidity exhibited by the comparison solution |
| Purity test (silicate) (%) | 1.5 or less | 0.5 |
| Purity test (heavy metal) | The color exhibited by the test solution is not darker than the color exhibited by the comparison solution. (20 ppm or less) | The color exhibited by the test solution is not darker than the color exhibited by the comparison solution (20 ppm or less) |
| Purity test (arsenic) | The test solution is not darker than the standard color. (2 ppm or less) | The test solution is not darker than the standard color (2 ppm or less) |
| Purity test (starch) | The liquid does not appear blue in color. | The liquid does not appear blue in color |

As shown in Tables 5 and 6 above, it can be seen that no toxic materials or microplastics were detected from the prepared CMC polymer, and the prepared CMC polymer is commercialized to meet all the criteria (confirmation test, purity test, and the like) of the Ministry of Food and Drug Safety for application in the human body.

**Preparation Example 3**

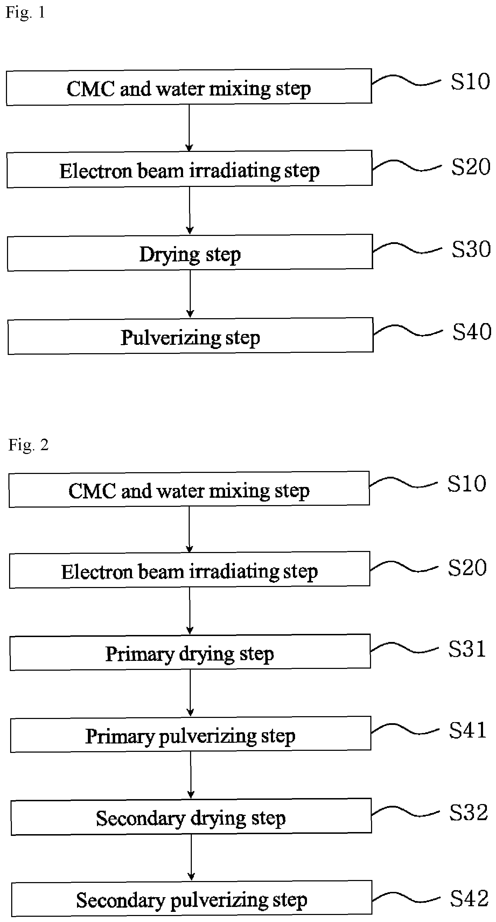

After 10 wt% of a CMC with a viscosity of 1500 cps was mixed with 90 wt% of water, a plurality of samples were irradiated with different doses of electron beams as shown in FIG. 1 to induce a crosslinking reaction. The crosslinked mixture was dried at 40°C until being completely dried, and then pulverized to prepare particles with an appropriate particle size. (Examples 3-1 to 3-24)

**Experimental Example 3**

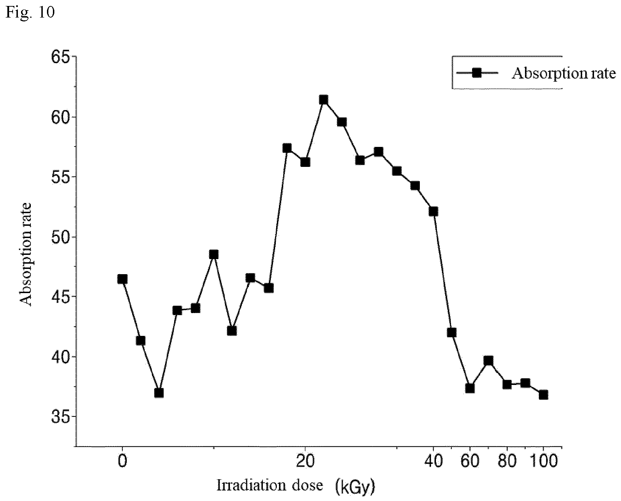

The absorption rate of 1 wt% of brine was evaluated for the CMC polymers prepared in Examples 3-1 to 3-24 using the following method, and the results are shown in FIG. 10.

- Absorption rate: KSP ISO 17190-5 : 2001, mass fraction (g/g)

As shown in Figure 10, it can be seen that a CMC polymer with the best absorption rate may be prepared when the irradiation dose is set to 18 to 40 kGy because the absorption rate increases rapidly at an irradiation dose of 18 kGy or more whereas it decreases rapidly at an irradiation dose of 40 kGy or more.

As described above, although the present invention is mainly described with reference to the embodiments of the present invention, this is merely an example and does not limit the present invention, and it will be appreciated that a person with ordinary skill in the art to which the present invention pertains can make various modifications and applications which are not exemplified above within a range not departing from the essential characteristics of the embodiments of the present invention. For example, each constituent element specifically shown in the embodiments of the present invention can be modified and implemented. And differences related to these modifications and applications should be construed as being included in the scope of the present invention defined in the appended claims.

The eco-friendly super absorbent CMC polymer according to embodiments of the present invention can have very good biodegradability. Further, the eco-friendly super absorbent CMC polymer is suitable for use in sanitary products such as sanitary pads and diapers because it is free of toxic materials or microplastics and has high absorbency and appropriate gel strength.

In addition, the method for preparing a CMC polymer of the present invention is excellent in process efficiency such as time taken for drying and pulverization yield compared to the physical properties of the CMC polymer.

The effects according to the embodiments of the present invention are not limited to the contents exemplified above, and more various effects are included in the present specification.

**Claims**

1. A method for preparing an eco-friendly super absorbent carboxymethyl cellulose (CMC) polymer, the method comprising:

   (a) mixing 5 to 25 wt% of a CMC with a viscosity of 1,000 cps or more and 75 to 95 wt% of water;
   (b) irradiating the mixture with an electron beam;
   (c) drying the irradiated mixture at 10 to 70°C; and
   (d) pulverizing the dried mixture.

2. The method of claim 1, wherein the drying temperature is a temperature, such that a temperature dependent utility value ($F_2$) calculated by the following Equation 1-2 of the CMC polymer prepared by the method is 2.2 or more

$$[\text{Equation } 1\text{-}2]$$
$$F_2 = X_2 + Y + Z$$

in Equation 1-2, for each of $X_2$, Y and Z,

   i) $X_2$ = 1.2 (when the time taken for drying is 0 hour or more and less than 24 hours)

$X_2$ = 1 (when the time taken for drying is 24 hours or more and less than 48 hours)
$X_2$ = 0.9 (when the time taken for drying is 48 hours or more and less than 72 hours)
$X_2$ = 0.7 (when the time taken for drying is 72 hours or more and less than 96 hours) or
$X_2$ = 0.3 (when the time taken for drying is 96 hours or more)
ii) Y = y^2 (y is the biodegradability)
iii) Z = z (z is the pulverization yield).

3. The method of claim 1, wherein the irradiation dose of the electron beam is 10 to 40 kGy.

4. An eco-friendly CMC polymer prepared by the method of claim 1, wherein the eco-friendly CMC polymer satisfies one or more of a time taken for drying of 30 to 85 hours, a biodegradability of 0.6 to 0.9 and a pulverization yield of 0.8 to 0.99.

5. The eco-friendly CMC polymer of claim 4, wherein a temperature dependently utility value ($F_2$) calculated by the following Equation 1-2 is 2.2 or more

[Equation 1-2]

$$F_2 = X_2 + Y + Z$$

in Equation 1-2, for each of $X_2$, Y and Z,

i) $X_2$ = 1.2 (when the time taken for drying is 0 hour or more and less than 24 hours)
$X_2$ = 1 (when the time taken for drying is 24 hours or more and less than 48 hours)
$X_2$ = 0.9 (when the time taken for drying is 48 hours or more and less than 72 hours)
$X_2$ = 0.7 (when the time taken for drying is 72 hours or more and less than 96 hours) or
$X_2$ = 0.3 (when the time taken for drying is 96 hours or more)
ii) Y = y^2 (y is the biodegradability)
iii) Z = z (z is the pulverization yield).

Fig. 1

```
┌─────────────────────────────────────┐
│     CMC and water mixing step        │  ⌇── S10
└─────────────────────────────────────┘
                  │
                  ▼
┌─────────────────────────────────────┐
│    Electron beam irradiating step    │  ⌇── S20
└─────────────────────────────────────┘
                  │
                  ▼
┌─────────────────────────────────────┐
│            Drying step               │  ⌇── S30
└─────────────────────────────────────┘
                  │
                  ▼
┌─────────────────────────────────────┐
│          Pulverizing step            │  ⌇── S40
└─────────────────────────────────────┘
```

Fig. 2

```
┌─────────────────────────────────────┐
│     CMC and water mixing step        │  ⌇── S10
└─────────────────────────────────────┘
                  │
                  ▼
┌─────────────────────────────────────┐
│    Electron beam irradiating step    │  ⌇── S20
└─────────────────────────────────────┘
                  │
                  ▼
┌─────────────────────────────────────┐
│         Primary drying step          │  ⌇── S31
└─────────────────────────────────────┘
                  │
                  ▼
┌─────────────────────────────────────┐
│       Primary pulverizing step       │  ⌇── S41
└─────────────────────────────────────┘
                  │
                  ▼
┌─────────────────────────────────────┐
│        Secondary drying step         │  ⌇── S32
└─────────────────────────────────────┘
                  │
                  ▼
┌─────────────────────────────────────┐
│      Secondary pulverizing step      │  ⌇── S42
└─────────────────────────────────────┘
```

Fig. 3

Fig. 4

Fig. 5

Fig. 6

Fig. 7

Fig. 8

Fig. 9

Fig. 10

## INTERNATIONAL SEARCH REPORT

| International application No. |
|---|
| **PCT/KR2022/012619** |

| A. | CLASSIFICATION OF SUBJECT MATTER |
|---|---|

**C08B 11/12**(2006.01)i; **C08B 15/10**(2006.01)i; **C08J 3/28**(2006.01)i; **C08J 3/24**(2006.01)i; **C08L 1/28**(2006.01)i; **C08G 69/10**(2006.01)i; **C08L 77/04**(2006.01)i

According to International Patent Classification (IPC) or to both national classification and IPC

| B. | FIELDS SEARCHED |
|---|---|

Minimum documentation searched (classification system followed by classification symbols)

C08B 11/12(2006.01); A61L 27/00(2006.01); B01F 13/06(2006.01); C08B 15/00(2006.01); C08B 15/10(2006.01); C08J 3/24(2006.01); C08J 3/28(2006.01); C08J 9/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 카르복시메틸 셀룰로오스(carboxymethyl cellulose, CMC), 고흡수성 수지(super absorbent polymer, SAP), 전자빔(electron beam), 건조(dry)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2001-002703 A (JAPAN ATOM ENERGY RES. INST. et al.) 09 January 2001 (2001-01-09) See abstract; paragraphs [0019]-[0035]; and claims 1-11. | 1-5 |
| Y | KR 10-1778826 B1 (SHIMIZU, Takeshi et al.) 14 September 2017 (2017-09-14) See abstract; paragraphs [0010]-[0036]; and claims 1-7. | 1-5 |
| A | JP 2004-536624 A (PHILLIPS HYDROCOLLOIDS RES. LTD.) 09 December 2004 (2004-12-09) See abstract; and claims 1 and 32. | 1-5 |
| A | JP 2009-179706 A (SHIMIZU, T. et al.) 13 August 2009 (2009-08-13) See abstract; paragraphs [0030]-[0038]; and claims 1-6. | 1-5 |
| A | SHIN, H. K. et al. Role of electron beam irradiation on superabsorbent behaviors of carboxymethyl cellulose. Res Chem Intermed. 2014 [Published online: 19 August 2014], vol. 41, pp. 6815-6823. | 1-5 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 December 2022** | **08 December 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2022/012619** |

### C.     DOCUMENTS CONSIDERED TO BE RELEVANT

| Category* | Citation of document, with indication, where appropriate, of the relevant passages | Relevant to claim No. |
| --- | --- | --- |
| PX | KR 10-2432556 B1 (INERTIA INC.) 16 August 2022 (2022-08-16)<br>    See abstract; and claims 1, 3 and 4.<br>    *Published patent of a priority application of the present PCT application. | 1-5 |

Form PCT/ISA/210 (second sheet) (July 2022)

**INTERNATIONAL SEARCH REPORT**
**Information on patent family members**

International application No.

**PCT/KR2022/012619**

| Patent document cited in search report | | | Publication date (day/month/year) | Patent family member(s) | | | Publication date (day/month/year) |
|---|---|---|---|---|---|---|---|
| JP | 2001-002703 | A | 09 January 2001 | JP | 4819984 | B2 | 24 November 2011 |
| KR | 10-1778826 | B1 | 14 September 2017 | CN | 103025763 | A | 03 April 2013 |
| | | | | CN | 103025763 | B | 24 June 2015 |
| | | | | JP | 5416709 | B2 | 12 February 2014 |
| | | | | TW | 201141918 | A | 01 December 2011 |
| | | | | TW | I496821 | B | 21 August 2015 |
| | | | | WO | 2011-145216 | A1 | 24 November 2011 |
| JP | 2004-536624 | A | 09 December 2004 | CA | 2440863 | A1 | 19 September 2002 |
| | | | | EP | 1565483 | A2 | 24 August 2005 |
| | | | | RU | 2003130092 | A | 27 March 2005 |
| | | | | RU | 2280038 | C2 | 20 July 2006 |
| | | | | US | 2003-0027883 | A1 | 06 February 2003 |
| | | | | US | 2004-0059097 | A1 | 25 March 2004 |
| | | | | US | 6610810 | B2 | 26 August 2003 |
| | | | | US | 6841644 | B2 | 11 January 2005 |
| | | | | WO | 02-072862 | A2 | 19 September 2002 |
| | | | | WO | 02-072862 | A3 | 30 June 2005 |
| | | | | ZA | 200307398 | B | 06 July 2004 |
| JP | 2009-179706 | A | 13 August 2009 | None | | | |
| KR | 10-2432556 | B1 | 16 August 2022 | None | | | |

Form PCT/ISA/210 (patent family annex) (July 2022)